# EUROPEAN PATENT APPLICATION

(11) **EP 1 779 830 A1**
(43) Date of publication of application: **02.05.2007**
(21) Application number: 05023801.3
(22) Date of filing: 01.11.2005
(51) Int. Cl.: A61J 1/00, B65B 3/00

(54) **Drug transfer device and method**

(71) Applicant: Ingenium Universal ApS, 3400 Hillerød (DK)
(72) Inventor: Grotner, Jimmy, 3400 Hillerod (DK)

(57) **Abstract**

A drug transfer device includes a vertically movable vial clamp for holding vials of various sizes in position with their septum facing downwards, a syringe holder and actuator that includes a clamp for grasping the plunger of the syringe and a movable tray for supporting a bag containing an infusion solution. The syringe holder is movable between an aspiration position below the vial clamp with the syringe or the needle attached thereto pointing towards the septum of the vial and an expelling positioning in which the syringe or the needle attached thereto is pointing towards an injection port of the infusion bag. When the vial and the syringe are in place with the syringe holder in the aspiration position the vial clamp moves downwards so that the needle on the syringe pierces the septum. The actuator withdraws the plunger to a predefined extent thereby aspirating the desired amount of drug. Then, the vial clamp moves up, thereby disengaging the needle. This event is followed by the syringe holder moving to the expelling position. Then, the tray moves towards the syringe holder until the needle penetrates the injection port. Next, the actuator depresses the plunger, and the drug is expelled into the bag whereafter the tray is moved away from the syringe to disengage the needle.

## Description

### TECHNICAL FIELD

The present invention relates to devices and methods for handling drugs, in particular cytostatic drugs, and similar medicaments in connection with the transfer of a liquid drug from a vial to a bag filled with a solution for infusion.

### BACKGROUND ART

In hospitals around the world thousands of infusion bags filled with a mixture of drugs and a solution for infusion are prepared manually. In case of cytostatic drugs, many precautions need to be taken to secure absolute correct dosing of the drugs and to protect the staff and the environment from the extremely toxic drugs.

Therefore, preparation of infusion bags with cytostatic drugs is performed by a team of two pharmacists in accordance with a protocol. One of the pharmacists is the operator and the other is the controller. The operator wears protective clothing, a mask and gloves and sits at a laminar flow safety cabinet for protection from toxic fumes. The controller sits at a computer, typically a PC, within a distance that allows the team members to communicate orally with one another.

Inside the safety cabinet there is a display showing the same information as the display of the computer of the operator. At the start of the procedure the data for a patient are retrieved on the PC by the operator. Both displays will show the drug or drugs that have been prescribed for this patient.

The controller reads out the patient's name and the drugs that have been prescribed. The operator confirms these data. The prescribed drug or drugs that are contained in one or more vials are placed in a tray and the content of the tray is verified by the controller and then passed on to the operator.

The operator also verifies the content of the tray against the information on the display in the safety cabinet and prepares a number of disposable syringes equal to the number of vials in the tray.

The controller instructs the operator to take a first (or only) vial and to aspirate the prescribed amount of drug into the barrel of the syringe. The operator takes one of the disposable syringes with a hypodermic needle thereon and weighs the empty syringe on a digital scale inside the safety cabinet and the operator enters the reading of the scale in the PC. The operator and the controller also prepare an infusion bag. The weight of the infusion bag is also determined with the scale and the reading of the scale is entered by the controller in the PC.

Then, the operator pierces the septum of the first vial with the needle on the syringe and withdraws the plunger until the prescribed amount of drug is aspirated into the barrel of the syringe. Thereafter, the operator places the filled syringe on the digital scale and the reading of the scale and the controller enters the reading of the scale in the PC. The controller and the operator convert the weight difference of the syringe before and after the aspiration step and with the help of a calculator and a lookup table the corresponding volume in a weight to volume lookup table to compare the volume in the barrel of the syringe with the prescribed volume of the drugs to be added to the bag. If the volumes match, the operator pierces the injection port of the bag and injects the complete contents of the barrel of the syringe into the bag. If the volumes do not match the procedure is repeated from scratch.

The procedure is repeated until all the prescribed drugs have been injected in the infusion bag. Finally, the bag with the drugs therein is weighed again and the operator and the controller checks that the overall weight increase of the bag matches the overall amount of drugs that were supposed to be added to the bag. If these amounts match within a narrow range the operator enters this event in the PC and the PC prints a self-adhesive label for the bag on a label printer that is connected to the computer and the operator attaches the label to the bag. The vial and the syringes are disposed of in a container for toxic waste that is placed inside the safety cabinet.

A problem associated with the manual transfer is repetitive strain injury caused by the repetitive action of withdrawing and depressing the plunger of the syringe. The syringes can be of various sizes, and in particular the larger ones require a significant force to be applied to move the plunger. Also the action of penetrating the septum and in particular the injection port requires a force and precision. For the penetration of the injection port the situation is even worse since the plunger may not be touched to avoid any premature expelling of the drug, which means the operator may only hold the barrel.
Further, these actions require a precise control of the movement and therefore the rate of repetitive strain injuries amongst the operators is alarmingly high.
Further, the manual weighing of the syringes and the infusion bags is time consuming and not guaranteed error free.

### DISCLOSURE OF THE INVENTION

It is an object of the present invention to provide device for transferring a liquid drug from a vial to another medical container that will reduce the risk of repetitive stress injury for the operators. This object is achieved in accordance with the devices and methods described below.

According to claim 1 a drug transfer device comprises a substantially vertically movable vial clamp for holding vials of various sizes in position with the septum of the vial facing downwards, a movable tray for supporting an infusion bag that is provided with an injection port, a syringe holder for receiving a disposable syringe with a needle attached thereto, the syringe holder being movable between an aspiration position below the vial clamp with the needle pointing towards the septum of the vial and an expelling position which the needle is pointing towards the injection port of an infusion bag on the tray, and an actuator associated with the syringe holder, the actuator being provided with a clamp for grasping the plunger of the syringe.

Preferably, the vial clamp can move between an upper position in which the septum of a vial in the vial clamp is disposed above the tip of the needle of a syringe in the syringe holder in the aspiration position, and a lower position in which the septum of a vial in the vial clamp is penetrated by the tip of the needle of a syringe in the syringe holder in the aspiration position.

The movement of the vial clamp can effected by a first electric drive motor.

The device may further comprise an internal or external controller, wherein the controller is configured to control the movement of the vial clamp.

Preferably, the tray is moveable between a remote position in which the injection port of an infusion bag on the tray is disposed in front of the tip of the needle of a syringe in the syringe holder in the expelling position, and a proximate position in which the injection port of an infusion bag on the tray is penetrated by the needle of a syringe in the syringe holder in the expelling position.

The movement of the tray may be effected by a second electric drive motor.

The the controller can be configured to control the movement of the tray.

The actuator can be operated by a third electric drive motor, and the control unit is configured to control the movement of the actuator.

The controller can be configured to command the actuator to withdraw the plunger of the syringe when the syringe holder is in the aspiration position and the vial clamp is in the lower position.

The controller can also be configured to command the first electric drive motor to move the vial clamp from the lower position to the upper position' after the plunger has been withdrawn.

Preferably, the syringe holder with the actuator thereon is pivotally suspended from a frame or housing of the device about a substantially horizontally extending pivot axis that is disposed below the vial clamp with the aspiration position of the syringe holder being a substantially upright position and the expelling position being a non-upright position, preferably a substantially horizontal position, and the movement between the aspiration position and the expelling position being a pivotal movement about the pivot axis.

The movements of the syringe holder can be effectuated by a fourth electric drive motor, preferably under control of the controller.

The controller can be configured to move the syringe holder from the aspiration position to the expelling position after the plunger has been withdrawn.

The controller can also be configured to command the tray to move from the remote position to the proximate position after the syringe holder has moved from the aspiration position to the expelling position.

The controller can be configured to command the actuator to depress the plunger of the syringe when the syringe holder is in the expelling position and the tray is in the proximate position.

Preferably, the vial clamp is suspended from a weighing cell that is connected to the controller, and the controller is configured to determine the difference in weight of the vial before the drug has been withdrawn from the vial and after the drug has been withdrawn from the vial.

Preferably, the tray is suspended from a weighing cell that is connected to the controller, and the controller is configured to determine the difference in weight of the vial before the drug has been injected into the solution bag and after the drug has been injected into the solution bag.

The controller can be configured to compare the determined weight difference of the vial and/or the infusion bag with a predetermined desired amount of drug to be delivered to the bag.

Preferably, one or more of the first, second, third and fourth electric drive motors is provided with position or movement determining sensors, preferably encoders, and the signal of the sensors being supplied to the controller.

The controller can be a Programmable Logic Controller inside a housing of the device or be a computer connected to the device.

Preferably, the actuator includes a shaft driven by the third electric drive motor via a transmission, the shaft being concentric with the pivot axis and provided with a toothed pulley for driving a toothed belt, the toothed belt running around another pulley that is suspended from the syringe holder and the toothed belt being operatively connected to the actuator.

The syringe holder may include a spindle extending in parallel with the longitudinal axis of the syringe, the spindle is driven by the third electric drive motor via a transmission and the actuator is operably connected to a nut that is in threaded engagement with the spindle.

The vial clamp can be a diaphragm type clamp with at least three circularly arranged clamping shoes.

Preferably, the clamping shoes are resiliently urged towards an engaged position, preferably by permanent magnets, and can be manually moved to a disengaged position via a handle operated mechanism.

The tray may comprise a port clamp for fastening but not occluding the injection port.

The port clamp may include a wedge-shaped recess with a lumen below a neck.

Preferably, the syringe holder comprises a pivotally suspended funnel-shaped receiver for receiving the front end of the syringe, the funnel shaped receiver has a large opening in which the needle and the syringe are inserted and a small opening through which the needle of a syringe protrudes, the transition from the large opening to the small opening includes a conical inner wall with an angle that matches the angle of the conical front end of a standard syringe, the conical section preferably leading to a cylindrical section with a diameter substantially corresponding to the outer diameter of a standard Luer-lock.

The clamp of the actuator may includes a transverse slit for receiving a part of the circular plate that is disposed at the free end of the plunger, and an axial slit for receiving one of the walls of the cross shaped cross-section of the plunger rod.

The controller may be configured to move the actuator to a position in which the transverse slit is at a distance from the receiver that matches the size of the syringe that a user or operator has indicated to the controller via a user interface.

The receiver can be suspended between two substantially parallel rims that extend in the longitudinal direction of the syringe and are separated by a distance larger than the diameter of the largest syringe to be used.

Preferably, the two rims are provided with transverse slits for receiving the transverse flanges that extend in opposite directions from the end of the barrel of the syringe, preferably a plurality of these slits are disposed at various positions along the rims at a distance from the receiver that matches the various lengths of standardized syringes.

The device may further comprise a barcode scanner for reading a barcode provided on the vial and/or the infusion bag.

According to claim 3 a method for transferring a liquid drug from a vial into an infusion bag or bottle, comprising the steps of providing a vial holder and placing a vial in the vial holder, providing a syringe holder, and placing a syringe provided with a needle in the syringe holder, providing a bag or bottle holder, and placing a bag with an injection port or a bottle with a septum in the bag or bottle holder, providing an actuator for withdrawing and depressing the plunger of the syringe, placing the syringe holder with the needle in front of the septum of the vial, moving the syringe holder and the vial holder towards one another until the septum of the vial is penetrated by the needle on the syringe, aspirating the drug into the barrel of the syringe by the actuator withdrawing the plunger of the syringe, moving the syringe holder and the vial holder away from one another until the septum of the vial is disengaged from the needle on the syringe, placing the syringe holder with the needle in front of the injection port of the bag or in front of the septum of the bottle, moving the syringe holder and the bag or bottle holder towards one another until the injection port or the septum of the bottle is penetrated by the needle on the syringe, and expelling the drug from the barrel of the syringe by the actuator depressing the plunger of the syringe, thereby injecting the drug into the bag or bottle.

The drug transfer procedure may be controlled with a PLC in the device or with an external computer.

Preferably, the method further comprises the steps of: recording the weight of the syringe before and after the aspiration of the drug in a computer, and the computer determining the weight difference, the computer determining the drug volume corresponding to the determined weight difference and the computer comparing the determined drug volume with the prescribed volume.

The prescribed type of drug, the prescribed drug amount, and the effective syringe plunger surface can be stored in the computer or PLC controlling the transfer procedure

The method may further comprise the step of reading a barcode label associated with a drug vial and/or with an infusion bag and with a barcode reader that is operatively connected to the copter or PLC controlling the drug transfer method.

Preferably, the method further comprises the steps of: recording the weight of the infusion bag before and after the aspiration of the drug in a computer, and the computer determining the weight difference, the computer determining the drug volume corresponding to the determined weight difference and the computer comparing the determined drug volume with the prescribed volume.

According to claim 5 a holder for grasping a cap of a vial, comprises a housing with a substantially cylindrical recess for receiving the top of the vial, the bottom of the recess being provided with an aperture for allowing needle access to the top of a vial received inside the recess, three or more radially movable clamping members for engaging the top of the vial, the clamping members being circularly arranged around the recess, means for urging the clamping members toward the axis of the cylindrical recess and means for moving the clamping members away from the axis of the cylindrical recess.

According to claim 6 an actuator for a vial holder comprises a linear drive with a movable drive member, a vial holder with clamping means for engaging a top of a vial, the vial holder being suspended from the drive member, and a dynamic weighing cell disposed between the drive ember and the vial holder.

According to claim 7 a receiver for receiving the tip of a Luer-lock syringe comprises a funnel shaped housing a conical section leading to a substantially cylindrical section with a diameter substantially corresponding to a Luer-lock on a syringe.

According to claim 8 a syringe holder comprises two parallel rims provided with opposing pairs of slits and/or recesses for receiving the flanges on the end of the barrel of the syringe therein.

According to claim 9 a holder for holding the free end of the plunger of a syringe comprises a T-shaped slit, formed by a transverse slit for receiving a part of the thumbplate of the plunger and an axial slit for receiving a part of one of the four rims that make up the plunger rod.

According to claim 10 a tray for holding an infusion bag, the tray comprises means for holding the injection port of infusion bag, an actuator for effecting a translative movement of the tray relative to a bas of the tray in the direction of the injection port and a dynamic weighing cell disposed between the base and the tray.

### BRIEF DESCRIPTION OF THE DRAWINGS

In the following detailed portion of the present description, the invention will be explained in more detail with reference to the exemplary embodiments shown in the drawings, in which
Figure 1 is a perspective view on a device according to the invention in its operating environment,
figure 2 is a perspective view of a device according to a preferred embodiment of the invention in an initial state of a drug transfer procedure,

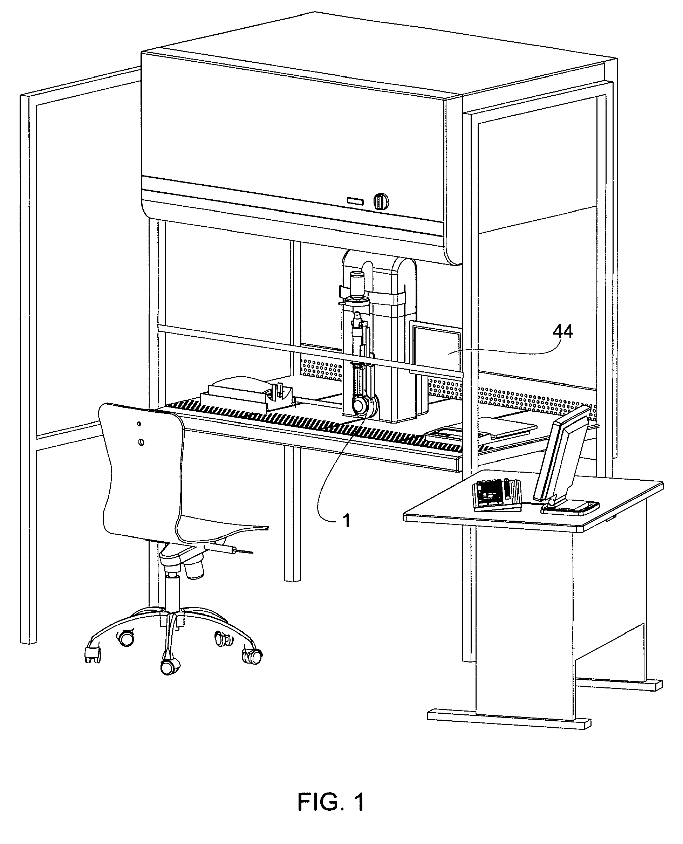

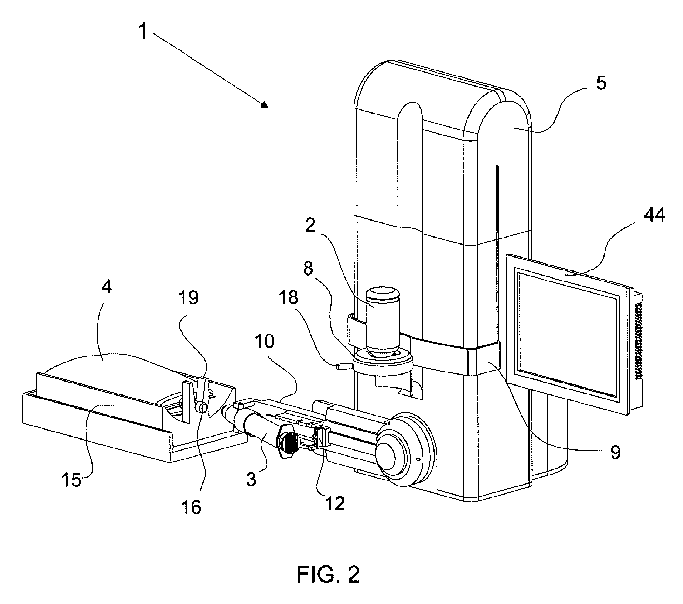

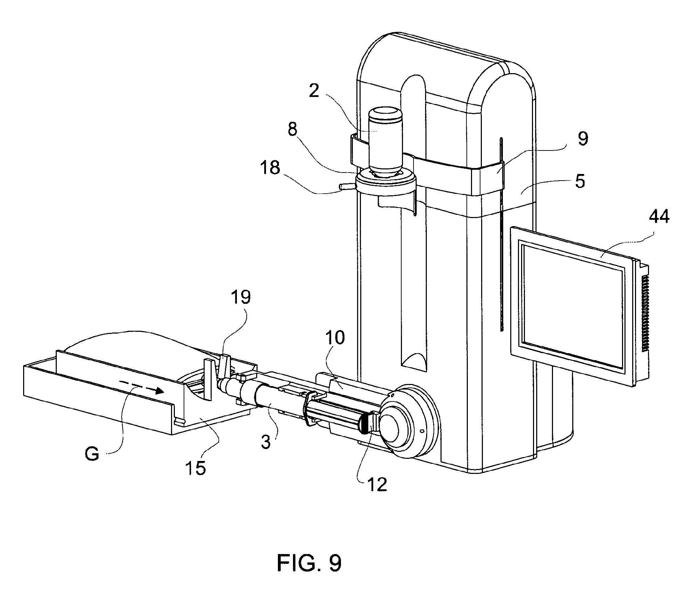

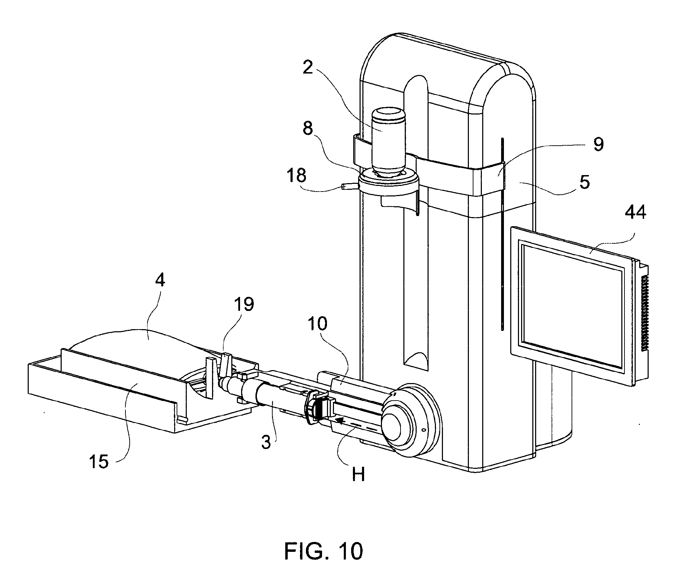

figures 2 to 10 are perspective views of the device of figure 1 in further states of the drug transfer procedure,

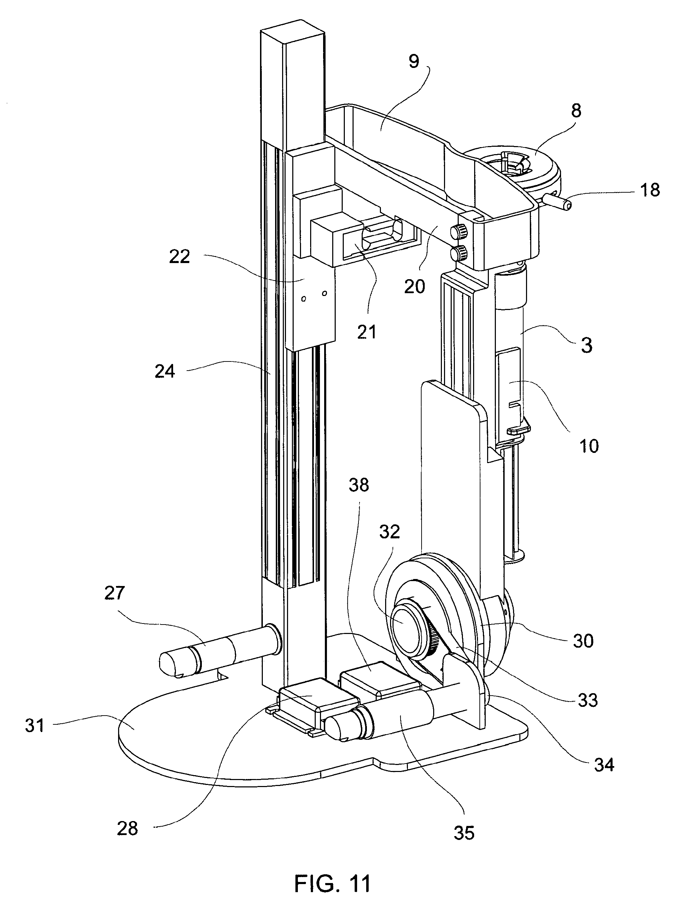

figures 11 to 14 are cut open perspective views of the device of figure 1 from various viewpoints,

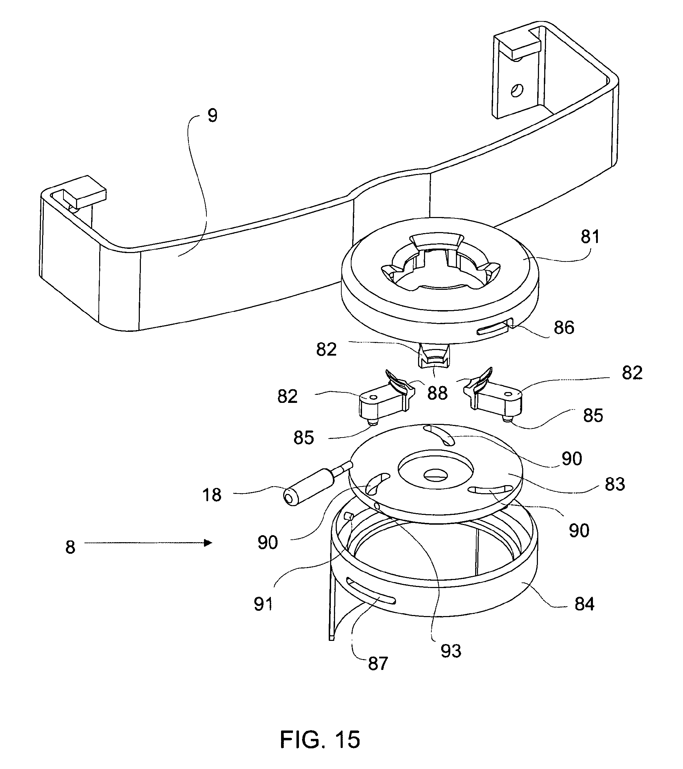

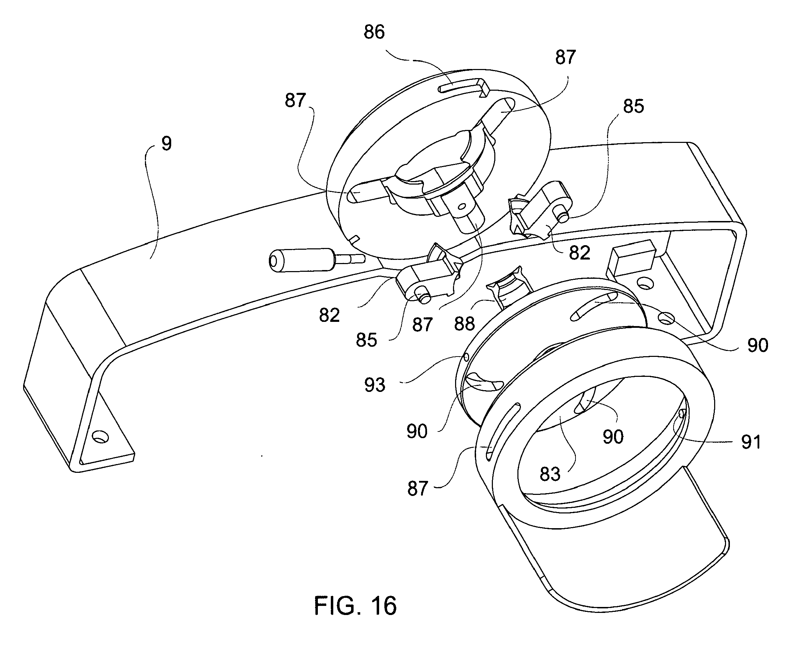

figures 15 and 16 are exploded views from different viewpoints of a vial holder according to the invention,

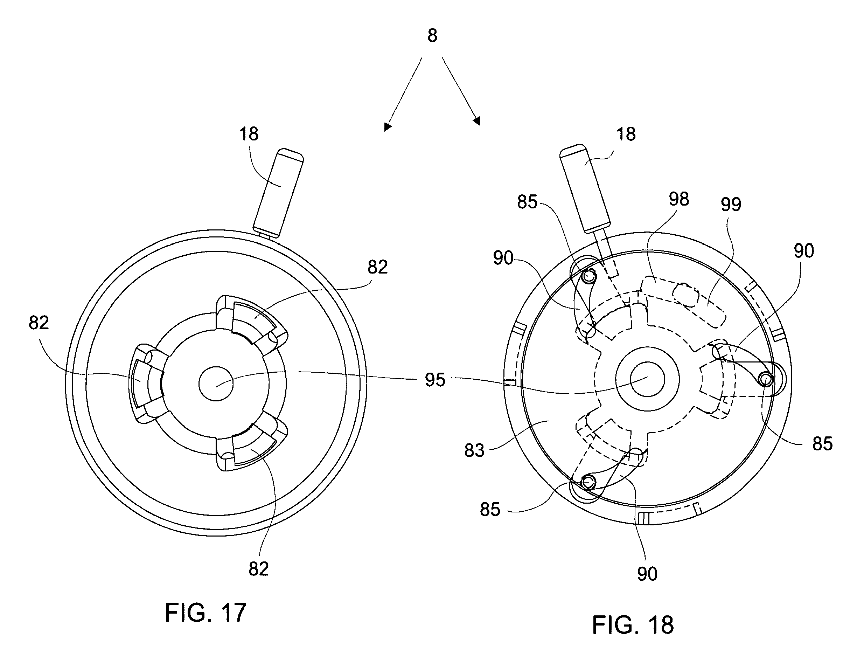

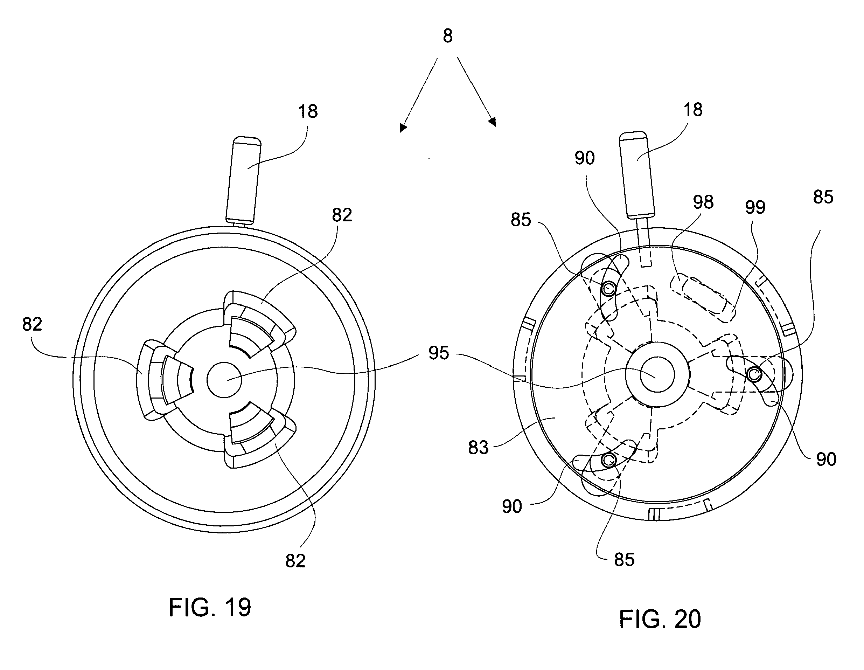

figures 17 to 22 are top and bottom views illustrating the operation of the vial clamp shown in figures 15 and 16,

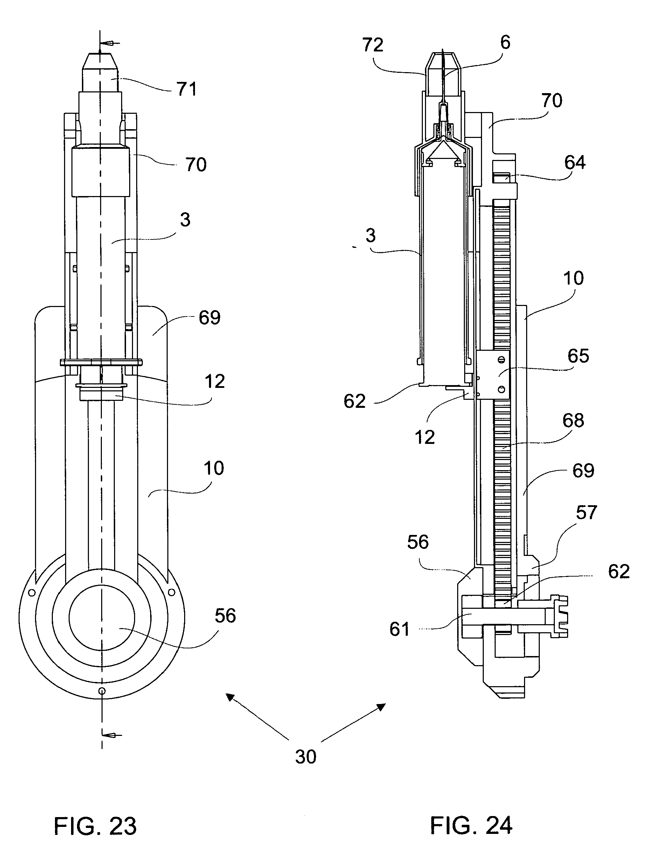

figure 23 is a side view on a syringe holder according to the invention,
figure 24 is a cross sectional view along line W-W' in figure 23,

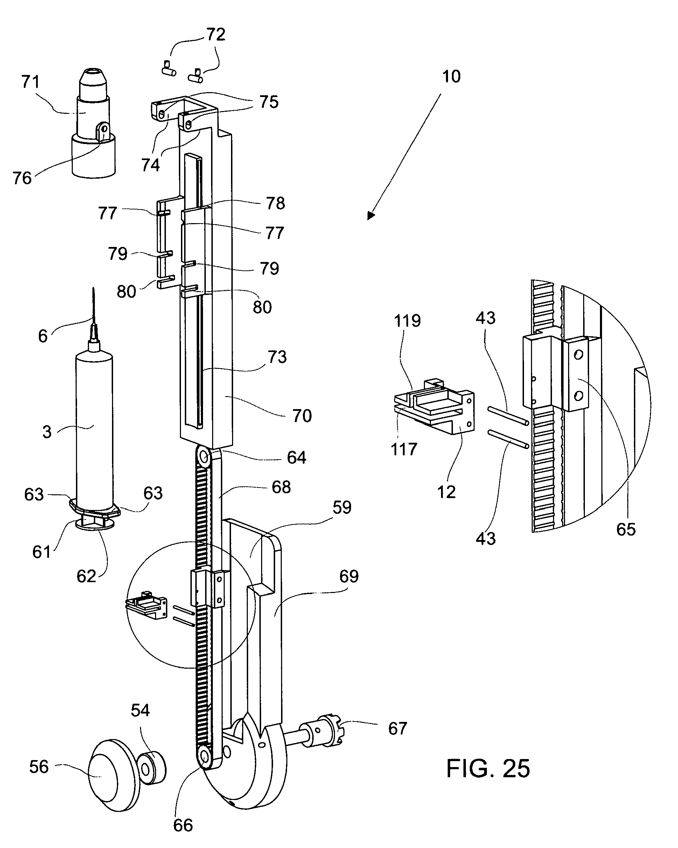

figure 25 is an exploded view of a first embodiment of the syringe holder shown in figures 23 and 24 and includes a detailed perspective view of an actuator,

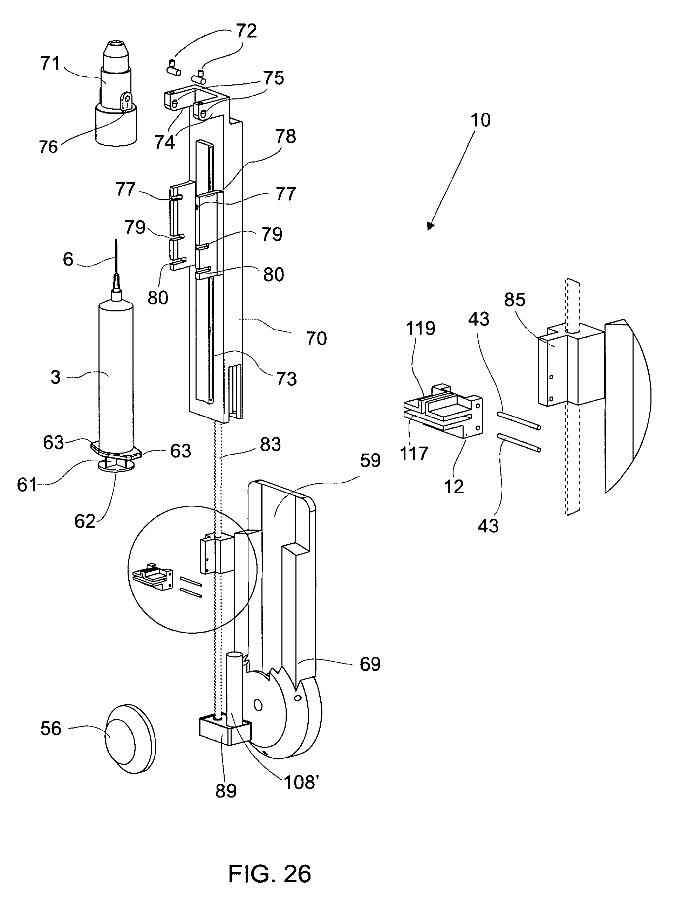

figure 26 is an exploded view of a second embodiment of the syringe holder shown in figures 23 and 24,

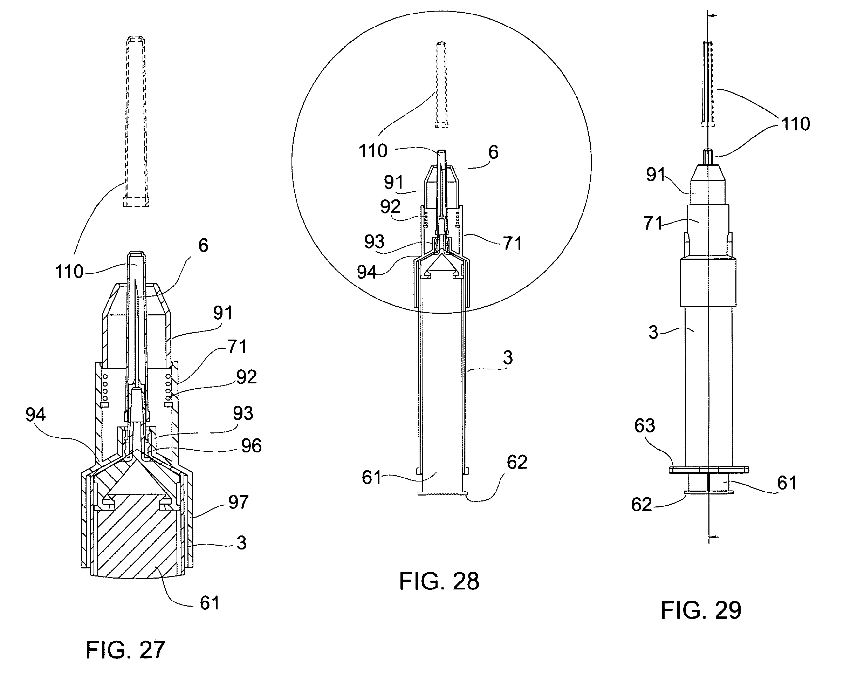

figures 27 and 28 are cross-sectional views of a syringe receiver according to the invention,
figure 29 is a side view of the syringe receiver shown in figures 27 and 28,

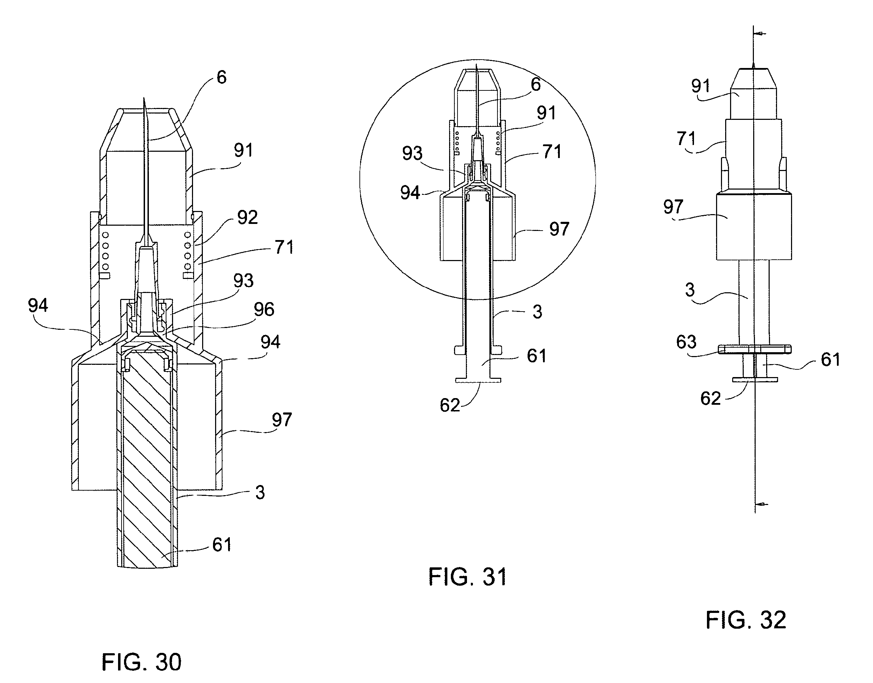

figures 30 to 32 illustrate the syringe receiver of figures 27 to 29 with a smaller syringe received therein,

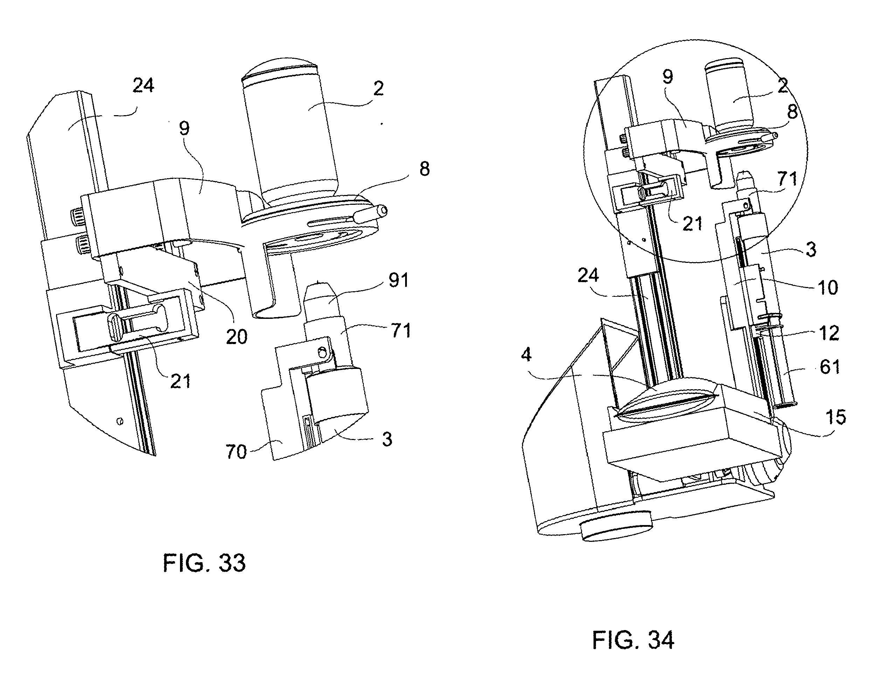

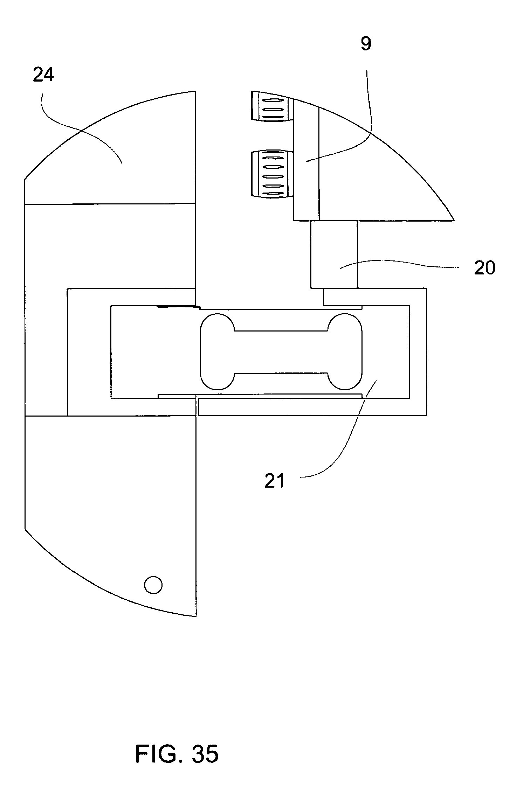

figure 33 and 34 are enlarged sections of figure 33 that illustrate a dynamic weighing cell used for weighing the vial holder,

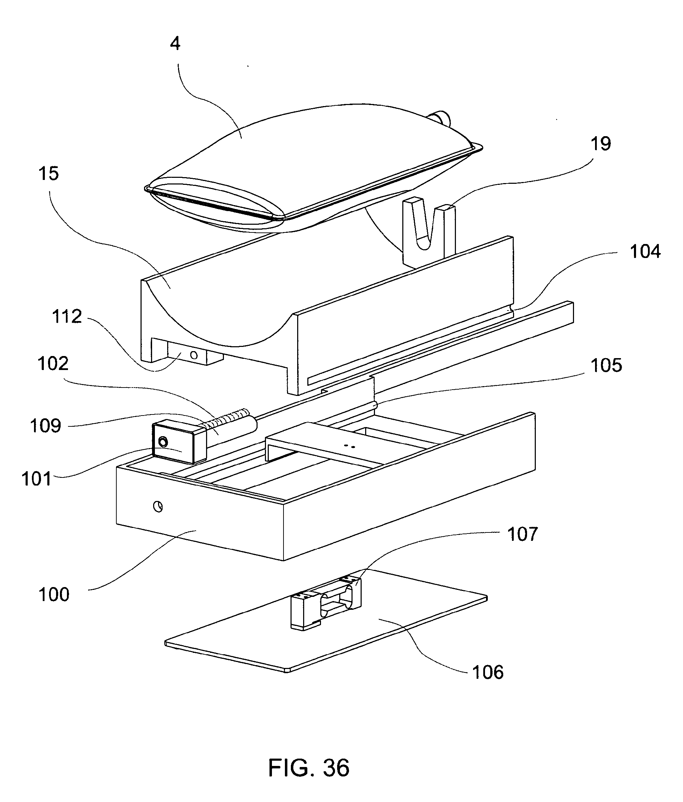

figure 36 is a perspective exploded view of a tray for receiving a solution bag, and

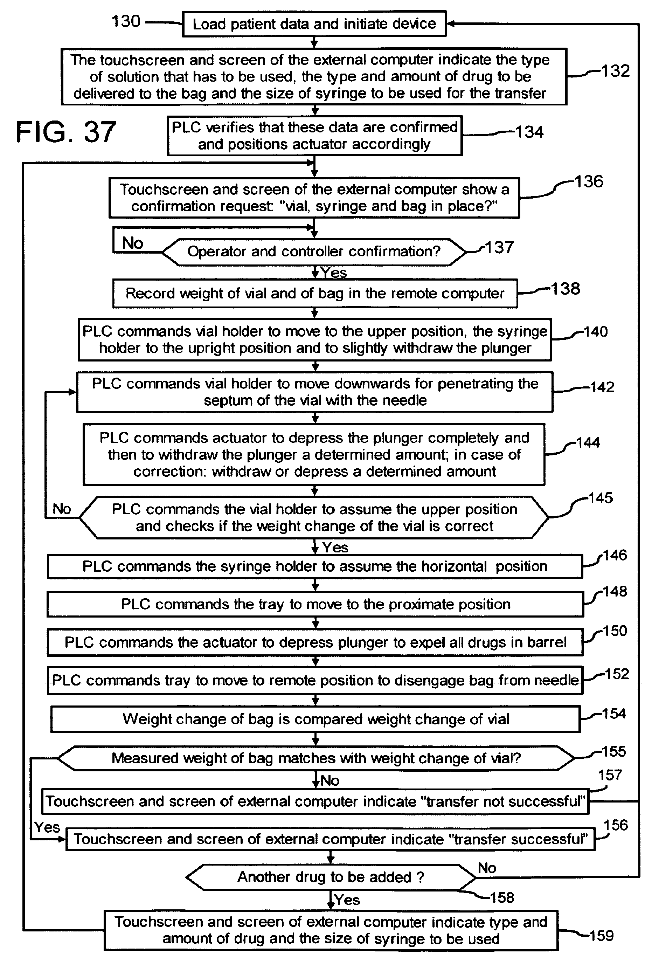

figure 37 is a flowchart illustrating the drug transfer procedure and the operation according to a preferred embodiment of the invention.

### DESCRIPTION OF THE PREFERRED EMBODIMENTS

Turning now to the drawings in greater detail, there is illustrated in figure 1 a laminar flow safety cabinet in which the device 1 according to a first preferred embodiment of the invention is placed. The safety cabinet ensures that any cytostatic or other poisonous drugs that are spilled and fumes thereof are contained in the safety cabinet and removed therefrom. The operator (not shown) sits in front of the safety cabinet, which has an opening for the arms of the operator in the lower part of the front of the cabinet. An external computer (cannot be seen since it hangs under the desk), such as a PC with a display, keyboard and mouse for use by the controller (person not shown), is placed in the vicinity of the safety cabinet. The external computer is connected to a server with patient data via a network. A scale for double checking weight measurements can also be present in the safety cabinet. Both the scale and the drug transfer device 1 are connected to the external computer. A touch screen 44 is suspended from the side of the drug transfer device 1. The touchscreen 44 is also connected to the external computer and shows the same information as the display of the external computer.

With reference to figures 2-9 the drug transfer device 1 and its operation is described in greater detail. The purpose of the device 1 is to transfer a liquid drug from a vial 2 into a disposable syringe 3 and to inject the drug from the disposable syringe 3 into a medical fluid container 4. The disposable syringe is typically a three component Luer-lock syringe. The container 4 can be a bag that is used for holding infusion solutions or alternatively transfusion solutions.

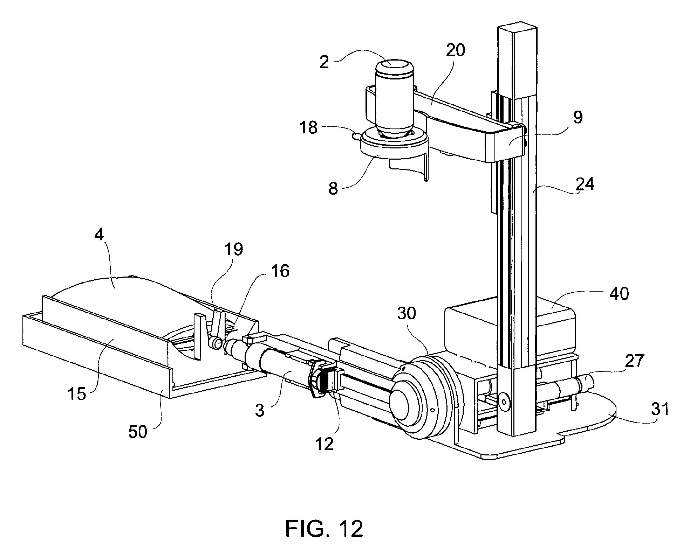

The operation of the device is controlled by a programmable logic controller (PLC) 40 (shown in figure 12), or by a control card in the external computer. The embodiments below are described with reference to the use of a PLC that is arranged inside the device 1. However, it should be understood that the control could be carried out by an external controller, such as a control card in an external PC. Data from a central computer or service of e.g. a hospital or other medical organization is used for controlling the operation of the device 1, and data produced by the device 1 is automatically recorded in the remote PC and can be uploaded to the central computer (not shown). Therefore, there is a wired or wireless connection between the PLC 40 and the external computer.

The drug transfer device 1 includes a housing 5, a vertically movable vial clamp 8, a pivotally movable syringe holder 10, an actuator 12, and a movable tray 15 for receiving the infusion bag 4. The housing 5 is elongated and extends with its longitudinal axis in an upright direction, i.e. in the direction of the laminar and flow in the safety cabinet so that the housing has only a minimal disturbing effect on the laminar airflow in the safety cabinet.

After an infusion bag 4 has been placed on the tray 15 a dynamic weighing cell (described in greater detail below) measures the weight of the infusion bag 4 before receipt of the drug(s) and the measured weight is recorded in the PLC 40 and/or in the remote computer.

The vial clamp 8 is carried by a vial clamp carrying bracket 9 that is connected to a linear drive member in the housing 5 via upright slits 14 in opposite sides of the housing 5. Thereby, it is avoided that any drugs that are spilled in the vicinity of the vial clamp 8 can enter the housing 5.

In figure 2 the vial clamp is in its lowest position so that it can be easily reached through the relatively low opening of the safety cabinet. The vial clamp 8 can be opened by rotating a handle 18 over an acute angle by a mechanism inside the vial clamp that will be described in greater detail below. After manually opening the vial clamp 8, a vial 2 is placed therein upside down, i.e. with the septum of the vial 2 facing downwards into the vial clamp 8. Then, the handle 18 is released and the vial clamp 8 engages the cap of the vial 2.

A dynamic weighing cell (described in greater detail below) measures the weight of the vial 2 before extraction of the drug and the weight reading is automatically recorded in the PLC 40 and/or the external computer.

A disposable syringe 3 with a hypodermic needle secured thereto is placed in the syringe holder 10 with the free end of the plunger (thumbplate) engaging the actuator 12. An infusion bag 4 is placed on the tray 15 with the injection port 16 of the bag 4 secured in an injection port clamp 19. The injection port clamp 19 fastens the injection port 16 without occluding the port. At this time the tray 15 is in its remote position, which means that the injection port 16 is at some distance from the tip of the needle on the disposable syringe 3.

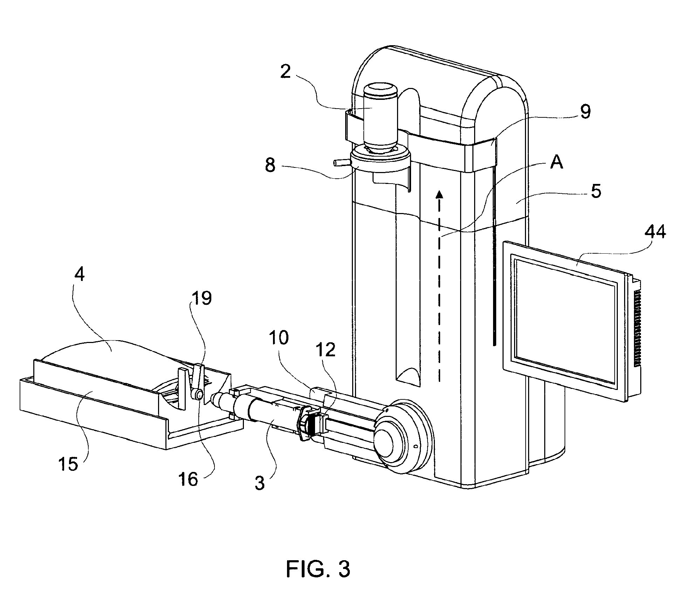

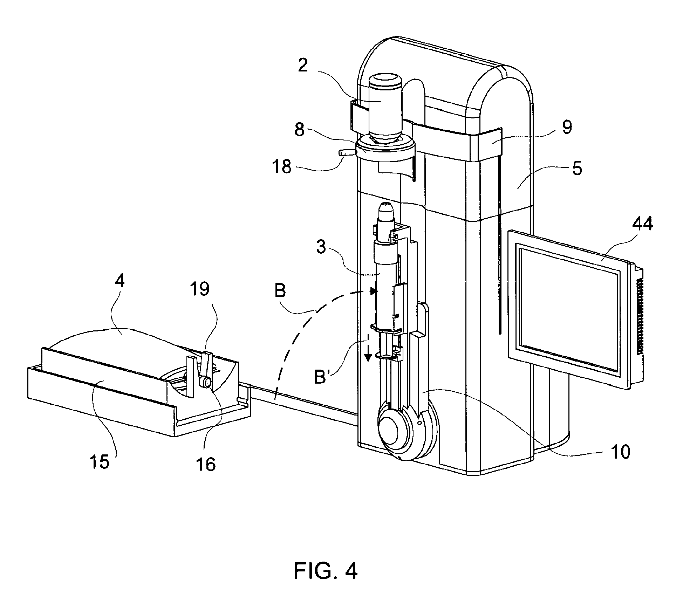

Next, as indicated in figure 3 by arrow A, the vial clamp 8 moves from the lowest position to the highest position. Then, the syringe holder 10 pivots as indicated by arrow B from the horizontal "expelling" position to the upright "aspiration" position (figure 4). At the same time the actuator 12 withdraws the plunger of the syringe 3 over a small distance to aspirate a small amount of air into the barrel of the syringe 3 - as indicated by arrow B'.

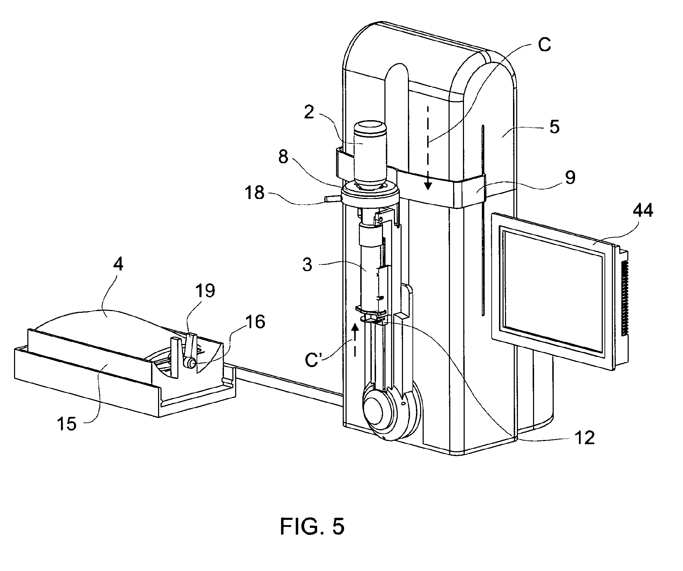

In the next step (figure 5) the vial holder 8 moves downwards as indicated by arrow C, whereby the tip of the needle pierces the septum of the vial 2. Then, the actuator 12 depresses the plunger of the syringe over a small distance (indicated by arrow C') to pump the small amount of air in the barrel of the syringe 3 into the vial 2, thereby slightly over-pressurizing the vial 2. The slight overpressure in the vial 2 facilitates the subsequent withdrawal of the drug from the vial.

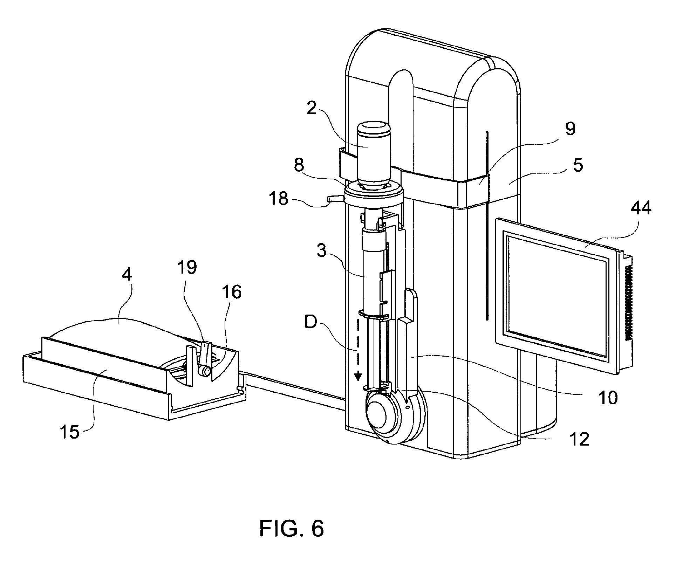

Next, as shown by arrow D in figure 6, the actuator 12 withdraws the plunger of the syringe to aspirate a desired amount of drugs from the vial 2. The speed with which the plunger is withdrawn is limited to about 1-1,5 ml/s. The PLC 40 takes account for the diameter of the syringe 3 for determining the speed with which the plunger is withdrawn so that the flow rate with which the drug is aspirated does not exceed 1-1,5 ml/s. The flow rate is limited to avoid turbulence and froth formation in the drug, which is highly undesirable.

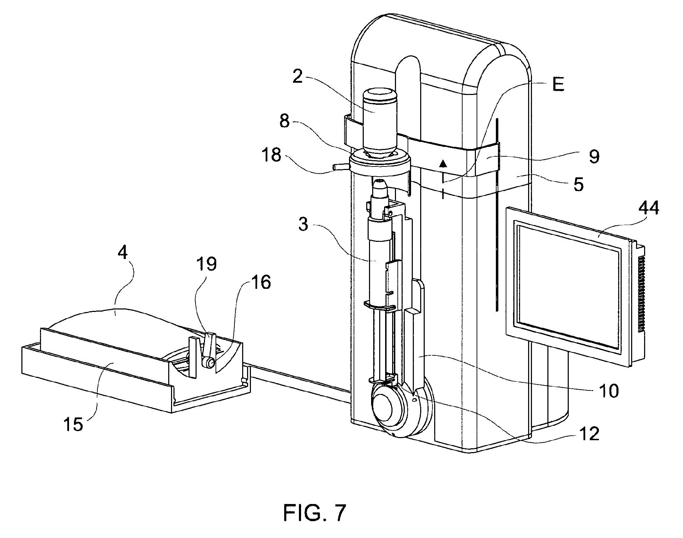

After the aspiration of the drugs into the barrel of the syringe 3, the vial clamp 8 moves upwards to the upper position as indicated in figure 7 by arrow E in order to disengage the septum from the needle. The weight of the vial 2 is after extraction of the drugs recorded in the PLC 40 and/or in the external computer. The weight change of the vial 2 is compared with the amount of drugs prescribed. When the weight change matches the prescribed amount of drug accurately enough, the device 1 moves to the next step. When the weight change of the vial 2 does not match accurately enough with the prescribed amount of drugs, the touchscreen 44 will indicate that the transfer was correct and the vial holder move down (not shown) to reengage the vial 2 with the needle for aspirating additional drug into the syringe 3 if there was not enough drug in the syringe 3 or for expelling drug into the vial 2 when there was too much drug in the syringe 3. After the correction procedure the amount of drug in the syringe is again verified by comparing the actual weight of the vial 2 with the original weight of the vial 2. Under normal circumstances, no further corrections should be required. If the desired amount of drug cannot be reached after three attempts the device indicate that there is an error and the procedure must be started form scratch, eventually after inspection of the device.

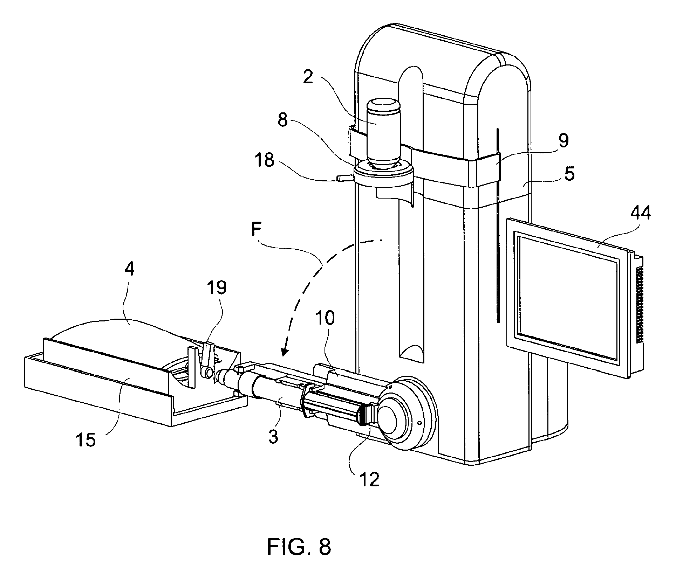

When the barrel is filled with the desired amount of drugs the syringe holder 10 is moved from the aspiration position to the expelling and position by a pivotal movement as shown in figure 8 by arrow F.

Then, the tray 15 moves from the remote position to the proximate position as indicated in figure 8 by arrow G. During this movement the needle on the syringe 3 penetrates the rubber plug in the injection port 16.

In the next step the actuator 12 depresses the plunger in the direction of arrow H, whereby the contents of the barrel are expelled therefrom and injected into the infusion bag 4. As with the aspiration, the flow rate during expelling is controlled not to exceed 1-1,5 ml/s. The flow rate is however not selected to be less than about 0,5 ml/s for both aspiration and expelling to ensure short cycle times.

After the drug has been injected into the infusion bag 4, the tray 15 moves from the proximate position to the remote position to disengage the syringe 3 from the injection port 16. The vial clamp 8 moves to its lowest position, and the device 1 has reached the state of figure 2 again. The weight of the bag 4 after receiving the drug is measured by a dynamic weighing cell in the tray and recorded in the PLC 40 and/or in the external computer. The weight changes of the vial 2 and of the bag 4 are compared, and when they match with a predetermined accuracy, the touchscreen 44 will indicate that the transfer was successful. When the weight change of the vial 2 and the bag 4 does not match accurately enough the touchscreen 44 will indicate that the transfer was not successful and the procedure starts all over again.

In the next step the operator removes the syringe 3 from the syringe holder 10 and places a new syringe 3 in the syringe holder 10. If required, the bag 4 is exchanged with a new bag 4. The vial clamp 8, the syringe holder 10, the actuator 12 and the tray 15 are now in the same position as shown in figure 2 and the cycle can be repeated to add another drug to the same infusion bag, or to inject a drug into a new infusion bag.

In the preferred embodiment the movements of the vial clamp 8, the syringe receiver 10, the actuator 12 and the tray 15 are effected by electric drive motors, transmissions and a controller that will be described in greater detail below. It is however noted that these movements could also be effectuated by appropriate handles and mechanisms operatively connecting the handles to the vial clamp, the syringe holder, the actuator and the tray.

Figures 11 to 14 show cut-away views on the interior components of the device 1. A linear drive 24 is disposed in an upright position on a base plate 31. The linear drive 24 is an off-the-shelf time drive (such as sold by the Swedish firm Rollco under the trademark Uniline A40), which is operated with a toothed belt that runs around toothed pulleys. Since these types of linear drives are well-known in the art the interior of the linear drive is not shown. The linear drive is powered by an electrical drive motor 27, which is powered by driver 28 that is controlled by the PLC 40 (shown in figure 12-14). The linear drive 24 includes a trolley 22 that can move along the linear drive 24 under the influence of the electric drive motor 27. A dynamic weighing cell 21 is attached to the trolley 22. A transverse horizontal bar 20 is attached to the free end of the dynamic weighing cell 21. A signal from the dynamic weighing cell is communicated to PCL 40. The clamp carrying bracket 9 is attached to the extremities of the transverse bar 20 by means of fasteners, such as bolts. Therefore, the load on the vial clamp 8 can be determined at any time.

The syringe holder 10 is pivotally suspended about hinge 30. The hinge has a substantially horizontal pivot axis and is disposed close to the base plate 31. The pivotal movement of the syringe holder 10 is affected by a drive mechanism that includes a toothed pulley 32 that moves in unison with the syringe holder 10. A toothed belt 33 is wrapped around pulley 32 and around a second toothed pulley 34. Toothed pulley 34 is driven by an electric drive motor 35. The electric drive motor 35 is powered by a driver 38, which is controlled by the PLC 40.

A further driver 48 powers a further electric drive motor (shown in figure 26) that is used for moving the actuator 12 (the details of this mechanism are described in greater detail further below).

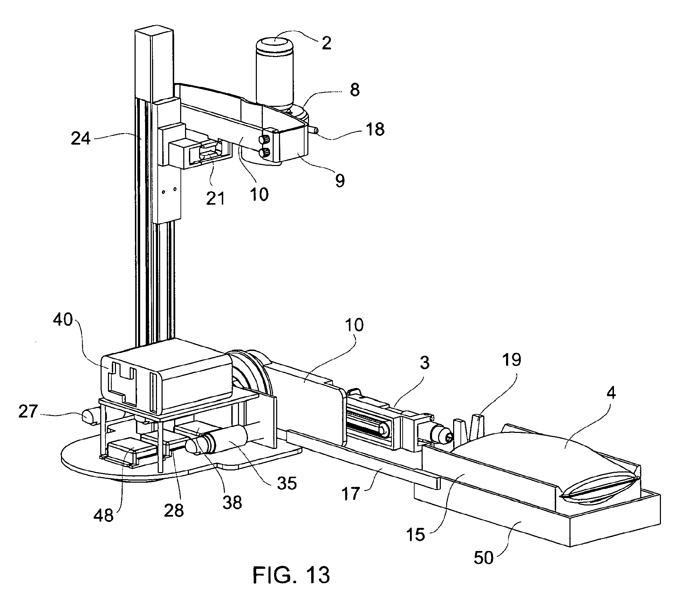

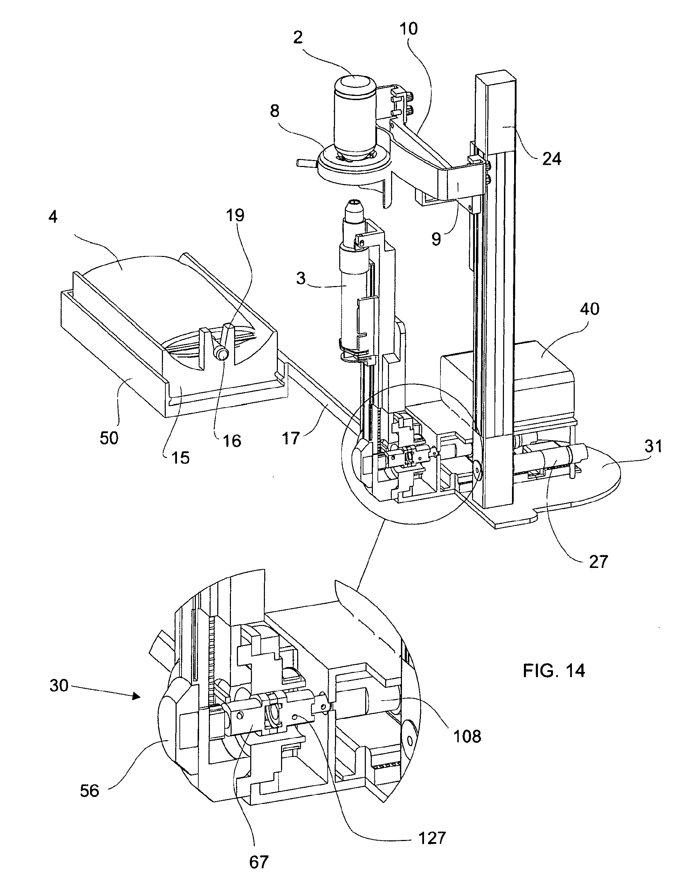

A bar 17 (figures 13,14) connects a base 50 of the tray 15 with the base plate 31 for ensuring that the distance between the tray 15, which is slidably suspended from the base 50, and the base plate 31 is fixed.

Figures 15 and 16 are exploded views in detail of the vial clamp 8 and the carrying bracket 9. The vial clamp 8 includes a circular housing cap 81 that is received in a substantially ring shaped housing 84. The housing cap 81 is secured to the ring shaped housing 84 by inwardly projecting radial pins 91 that engage L-shaped slits 86 formed in the circumferential wall of the housing cap 81. A rotatable disk 83 is received between the housing cap 81 and the ring shaped housing 84. The rotatable disk 83 is provided with a central aperture 95 to allow a needle 6 of the syringe to pass through the vial clamp 8. A pin extending from the handle 18 is received in a radial bore 93 in the rotatable disk 83. A tangential slot 87 in the ring shaped housing 84 allows the pin to extend through the radial wall of the ring shaped housing and provides for a limitation of the angular range of movement of the disk 83. Three clamping feet 82 provided with claws 88 are guided in radially disposed channels 87 formed in the housing cap 81. The clamping feet 82 are provided with downwardly projecting pegs 85. The pegs 85 are received in respective curved slots 90 that are formed in the rotatable disk 83.

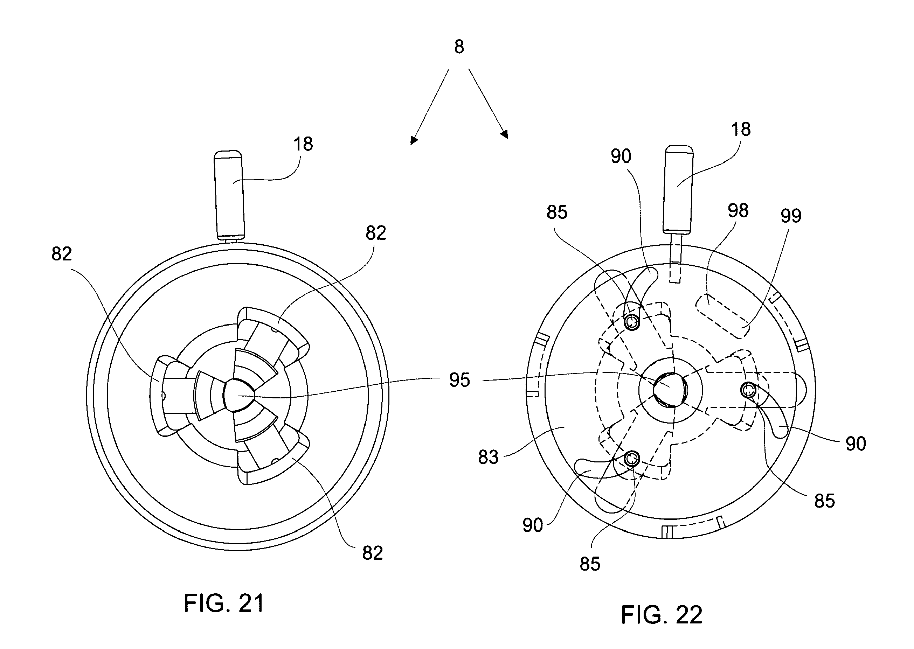

Figures 17 to 19 illustrate the opening and closing movement of the clamp feet 82 under the influence of the handle 18 and resilient means, such as permanent magnets 98,99 that urge the clamping feet 82 towards their closed position. Figures 17 and 18 are a top view and a bottom cross-sectional view of the vial clamp 8 with the clamping feet in the retracted position. A magnet 98 disposed in the housing cap 81 and a magnet 99 disposed in the rotatable disk 83 are positioned right above one another when the clamping shoes 82 are in the closed position as shown in figure 22. The poles of the magnets 98,99 are arranged such that the magnets attract one another and resists being moved away from one another.

When the rotatable disk 83 is a rotated anti-clockwise (anti-clockwise as in figures 18,20,22) from the position shown in figure 22 to the position shown in figure 18 the magnets are gradually moved away from another. Hereto, the considerable attractive force of the magnets 98 and 99 has to be overcome. This is achieved by a force applied by an operator on the handle 18. When the handle 18 is released the attractive magnetic force between the magnets 98 and 99 urges the rotatable disk to assume the position shown in figure 22. The interaction between the pegs 85 of the clamping shoes 82 and the curved slits 90 in the rotatable disk 83 urges the clamping shoes 85 towards the center of the vial clamp 8. In operation, the operator moves the handle to bring the clamping shoes 85 in their open position shown in figure 17 and 18. The interaction between the curved slots 90 and the pegs 85 causes the vial clamp to open and close in a manner similar to a diaphragm. The juxtaposed magnets 98,99 provide a relatively large force for urging the clamping feet as defined in the closed direction. When the operator releases the handle 18, the claws 88 of the clamping feet 82 engage the vial behind its cap, so that the vial 2 is securely fastened to the vial clamp 8 and the vial 2 will not disengage from the vial clamp 8 by the upwardly directed force applied to septum by the needle of the syringe.

Figures 23 to 25 show a syringe holder 10 according to a first preferred embodiment of the invention. The syringe holder 10 comprises a proximate part 69 and a distal part 70. The distal part received in a slot 59 (figure 25) formed in the proximate part 69. The proximate part 69 is suspended from its lower end around hinge 30, which includes a substantially horizontal axis that is concentric with a shaft 61. Shaft 61 is provided with two bearing rings 56,57, which are in turn received in circular recesses in the arm of the syringe holder 10, thus allowing the syringe holder 10 to pivot about shaft 61. A toothed pulley 62 is amounted on the shaft 61. Another toothed pulley 64 is rotably suspended from the upper end of the syringe holder 10 and a toothed belt 68 is wrapped around the pulleys 62,64. The actuator 12 is attached to the toothed belt 68 via a dog 65.

As shown in figure 25 and in the enlarged section of figure 14, the shaft 61 is connected via couplings 67 and 127 to an electric drive motor 88. Rotation of the shaft 61 effected by drive motor 88 results in a linear movement of the actuator 12. As shown in the enlarged section of figure 25, the actuator 12 is fastened to the dog 65 via dowel pins 43 or similar fasteners that pass through elongated axial slits 73 that are provided in the distal part 70. The fasteners, and thus also the slits 73 can be relatively thin, thus minimizing the area through which pollution could enter the interior of the syringe holder 10. The distal part 70 is at its upper end provided with a bracket 74. Bracket 74 pivotally suspends a funnel shaped syringe receiver 71 by means of pins 72 that are received in bores 75 that are disposed in the bracket 74 and engage bores 76 disposed in the syringe receiver 71. Syringe receiver 71 serves to receive the front end of the syringe 3. The barrel of the syringe 3 is received between two longitudinally disposed rims 77. The rims 77 are provided with recesses 78 and slits 79 and 80 for receiving the flanges 63 of the barrel of the syringe 3. The recesses 78 and the slits 79 and 80 are disposed at various distances from the syringe receiver 71 in order to be able to accommodate syringes 3 of various (standardized) sizes.

Figures 24 and 25 also show that the free end of the plunger 61 of the syringe is grasped by the actuator 12 by receiving the thumbplate 62 in a transverse slit 117 disposed in the actuator 12. An axial slit 119 (cf. enlarged section of figure 25) that is also disposed in the actuator 12 receives one of the four rims that make up the plunger rod 61. Together, the two slits 117,119 form a T-shaped slit. This arrangement for receiving the free end of the plunger 61 works well, since standard syringes are always packed with the rims that make up the plunger rod arranged such that two of the rims point at the flanges, with the other two rims at right angles to the flanges. When the flanges 63 of the syringe 3 are received in the recess 78 or in the slits 79,80, one of the rims of the plunger 61 will exactly fit into the axial slit 119 in the actuator 12. Thus, the plunger 61 is secured axially by the thump plate 62 being held by the transverse slit 117. The plunger 61 is secured transversely by one of the four rims that form the plunger rod 61 being received in the axial slit 119.

Figure 26 shows a second preferred embodiment of the syringe holder 10, wherein the proximate part 69 and the distal part 70 are substantially identical to the embodiment as described with reference to figure 25. However, in the second preferred embodiment the electric drive motor 88' is received within slot 59 and connected to a spindle 83 via a transmission 89. In this embodiment, the dog 65 is in threaded engagement with spindle 83. Activation of the electric drive motor 88' results in a translative movement of the dog 85 and the actuator 12.

Figures 27 to 29 show the syringe receiver 71 in greater detail. The syringe receiver 71 comprises a funnel-shaped housing with a wide body section 97 that has a large opening for receiving the front part of the syringe 3. The wide-body section 92 ends in a conical section 94, that matches the conical front part of the syringe 3, regardless of the size of the syringe. The conical section 94 connects to a cylindrical section 93. The diameter of the cylindrical section 93 is substantially the same as the outside diameter of the standardized Luer-lock of the syringe 3. The syringe receiver 71 is provided with a needle guard 91. The needle guard 91 is axially movable between an extended position in which most or all of the needle 6 is covered by the needle guard 91, and a retracted position in which most of the needle 6 is exposed. A helical spring 92 or other resilient means urges the needle guard 91 to the extended position. The syringe can be received in the syringe receiver 71 with its protective cap 110 in place, so that the protective cap may be removed (as indicated by the cap 110 shown with interrupted lines) after the syringe 3 has been received in the syringe receiver 71. In order for the operator to grab the cap 110, he/she depresses the needle guard 91 and pulls the protective cap 110 from the needle 6. During the cap removal procedure the needle guard 91 is urged upwards by the spring 92, and therefore the needle 6 is not exposed at any time.

Figures 30 to 31 illustrate how a smaller syringe 3 is held by the syringe receiver 71.

Figures 33 and 34, show the dynamic weighing cell 21 that is used to determine the change in weight of the vial 2 during the drug transfer procedure. One end of the dynamic weighing cell 21 is connected to the trolley 22, and the other end of the dynamic weighing cell 21 carries the transverse bar 20 that supports the bracket 9 with the vial clamp 8. The weighing cell generates a signal that is fed into the PLC 40.

Figure 36 shows the interior components of the tray 15. The tray is slidably mounted on a frame 50 via tracks 105 that protrude from the frame 50 and engage groves 104 that are disposed in the sides of the tray 15. The frame is connected to the base plate 31 by an arm 17 to ensure the correct distance between the tray 15 and the rest of the device 1. The movement of the tray 15 is effectuated by electric drive motor 109 that is controlled by the PLC 40. The electric drive motor 109 is coupled to a spindle 102 via a transmission 101. A nut 112 attached to the bottom of the tray 15 is in threaded engagement with spindle 102, so that rotational movement of the spindle under influence of the drive motor 101 results in a translative movement of the tray 15. The frame 15 is suspended from a bottom plate 106 by a dynamic weighing cell 107. The dynamic weighing cell 107 is connected to the PLC 40 and/or the external computer.

In a preferred embodiment the electric drive motors 27,35 109,88 and 88' are provided with encoders connected to the PLC 40 so that the latter receives a signal corresponding to the position of the times driven by the electric motors. The PLC 40 is provided with a port for communicating with an external computer. The PLC communicates the movements of the vial clamp 8, of the tray 15 of the syringe holder 10 and of the actuator 12 via the port to an external computer, so that the drug transfer procedure can be remotely monitored and can be recorded. The signals of the dynamic weighing cells 21 and 107 are also communicated from the PLC 40 to the external computer.

The device 1 as described with reference to the preferred embodiment can be provided with a barcode reader (not shown) connected to the PLC 40 and/or the external computer. A barcode reader will allow the type of drug and type of bag to be verified against a medical prescription for a patient.

Figure 37 shows a flowchart illustrating the procedure of transferring a drug from a vial 2 to a medical fluid container 4 using device 1. In step 130 the patient data and the medical prescription for the patient is loaded into the external computer (e.g. from a hospital server) and the data relevant for the device 1 - such as the type and amount of the drugs that need to be withdrawn from the one or more vials - are loaded into the PLC 40. Simultaneously, the PLC 40 initiates the device 1 by commanding the electric drive motors 27,35,88,101 to bring the vial holder 8, the tray 15, the actuator 12 and the syringe holder to assume the positions as shown in figure 2.

In step 132 the touchscreen 44 and the screen of the external computer indicate:
the type of solution bag 4 that has to be used the type and amount of the drug to be delivered to the bag; and
the size of syringe 3 to be used for the transfer.

In step 134 the PLC 40 verifies that these data are confirmed. If the device 1 is provided with a barcode reader, the PLC 40 awaits signals from the barcode reader that correspond to the barcode label on the bag 4 and on the vial 2. If there is no barcode reader, the operator must press on verification checkboxes on the touchscreen 44 and a controller (person) must mark verification checkboxes on the screen of the external computer that are assigned with any of the criteria above. Further, the PLC 40 moves the actuator 12 to a position that matches the position of the thumbplate 62 in dependence of the size of the syringe 3 used, i.e. closer to the syringe receiver 71 for smaller syringes and less close to the syringe receiver 71 for larger syringes 3.

In step 136 the touchscreen 44 and the screen of the external computer show a confirmation request that the syringe 3, the bag 4 and the vial 2 are correctly in place, and in step 137 the PLC 40 awaits a confirmation by the operator and the controller before proceeding further.

When the operator and controller confirmations have been received, the weight of the vial 2 and of the bag 4 is recorded in the external computer in step 138.

In step 140 the PLC 40 commands the vial holder 8 to move to the upper position and the syringe holder 10 to the upright "aspiration" position. Simultaneously, the PLC 40 commands the actuator 12 to withdraw the plunger 61 slightly.

In step 142, the PLC 40 commands the vial holder 8 to move downwards over a predetermined distance that is sufficient for penetrating the septum of the vial 2 with the needle 6.

In step 144 the PLC 40 commands the actuator to depress the plunger completely - thereby pumping the small amount of air in the barrel into the vial 2 - so that the vial 2 becomes slightly over-pressurized, and then the PLC 40 commands the actuator 12 to withdraw the plunger 61 over a length that corresponds to the amount of drug prescribed, thereby taking account for the diameter of the syringe 3 being used (the data of various syringe types, such as their length and plunger diameter, are stored in the PLC 40 and/or the external computer.

In step 145 the PLC 40 commands the vial holder to move to the upper position to disengage the vial 2 from the needle 6. The weight of the vial 2 is compared with its weight before the drug extraction. Since the prescription typically indicates a volume to be administered, tables or conversion factors stored in the external computer or in the PLC are used by the PLC 40 or the external computer to convert the measured weight to volume before a comparison is made. Then, the PLC 40 or the external verifies that the determined drug volume matches the prescribed amount of drug to be added accurately enough (typically within 0.5 ml). If the amount of drug in the barrel is correct, the procedure continues with step 146. If the amount of drug in the barrel is not correct the procure returns to step 142 to reengage the vial 2 on the syringe 6, in order to add or remove a required amount of drug to or from the syringe 3 (in step 144). When the correction is successful the procedure continues with step 146.

In step 146 the PLC 40 commands the syringe holder 10 to assume the horizontal "expelling" position.

In step 148 the PLC 40 commands the tray 15 to move to the proximate position for penetrating the injection port 16 of the bag 4 with the needle 6.

In step 150 the PLC 40 commands the actuator to depress the plunger 61 to expel all of the drugs in barrel of the syringe 3, thereby injecting the drug into the bag 4.

In step 152 the PLC 40 commands the tray to move to the remote position, thereby disengaging the bag 4 from the needle.

In step 154 the weight change of the bag 4 is compared with the weight change of the vial 2. The PLC 40 or the external computer verifies that the these weight change match one another accurately enough (typically within 0,5 g). If the weight changes match, the procedure continues with step 156. If the weight changes do not match the procedure continues with step 157.

In step 156 the touchscreen 44 and the screen of the external computer will indicate in step 156 that the transfer was successful. The PLC 40 or the external computer check in step 158 if the prescription includes a further drug to be added to the bag 4. If this is not the case the process returns to step 130 and the device 1 is ready for dealing with the next prescription. The vial 2 and the syringe(s) 3 are disposed off in a container for toxic waste that is placed inside the safety cabinet (not shown). If another drug is to be added to the bag 4, the touchscreen 44 and screen of external computer indicate type and amount of drug and the size of syringe to be used in step 159 and the process returns to step 136 to transfer the next drug in the prescription.

In step 157 the touchscreen 44 and the screen of the external computer will indicate in step that the drug transfer was not successful (an audible alarm may also be issued by the device 1 or the remote computer) and the process returns to step 130 to start over with a new bag 4.

Under circumstances that require fewer precautions, it is possible to operate the device 1 with an operator only, i.e. without a controller.

According to another embodiment of the device 1 (not shown), of the syringe holder 10 makes a translative movement between the aspiration position and the expelling position.

Further, not all movements of the device 1 need to be effectuated by electric drive motors. Instead handles operatively connected to the elements to be moved could be used.

Practically all components of the device 1 are preferably made from stainless steel, so that the whole device can be autoclaved. Dry running slide bearing materials with a running-in-layer of PTFE + ZnS, a sliding layer of CuSn10 with incorporated PTFE + ZnS and a steel backing are used between moving parts. Such bearing materials are commercially available from the German company KS Gleitlager under the trademark Permaglide®.

Although the present invention has been described in detail for purpose of illustration, it is understood that such detail is solely for that purpose, and variations can be made therein by those skilled in the art without departing from the scope of the invention.

## Claims

1. A drug transfer device for transferring a liquid drug from a vial to an infusion bag, said device comprising:
a substantially vertically movable vial clamp for holding vials of various sizes in position with the septum of the vial facing downwards,
a movable tray for supporting an infusion bag that is provided with an injection port,
a syringe holder for receiving a disposable syringe with a needle attached thereto, the syringe holder being movable between an aspiration position below the vial clamp with the needle pointing towards the septum of the vial and an expelling position which the needle is pointing towards the injection port of an infusion bag on the tray, and
an actuator associated with the syringe holder, the actuator being provided with a clamp for grasping the plunger of the syringe.

2. A device according to claim 1, wherein the vial clamp can move between an upper position in which the septum of a vial in the vial clamp is disposed above the tip of the needle of a syringe in the syringe holder in the aspiration position, and a lower position in which the septum of a vial in the vial clamp is penetrated by the tip of the needle of a syringe in the syringe holder in the aspiration position.

3. A method for transferring a liquid drug from a vial into an infusion bag or bottle, comprising the steps of:
providing a vial holder and placing a vial in the vial holder,
providing a syringe holder, and placing a syringe provided with a needle in the syringe holder,
providing a bag or bottle holder, and placing a bag with an injection port or a bottle with a septum in the bag or bottle holder,
providing an actuator for withdrawing and depressing the plunger of the syringe,
placing the syringe holder with the needle in front of the septum of the vial,
moving the syringe holder and the vial holder towards one another until the septum of the vial is penetrated by the needle on the syringe,
aspirating the drug into the barrel of the syringe by the actuator withdrawing the plunger of the syringe,
moving the syringe holder and the vial holder away from one another until the septum of the vial is disengaged from the needle on the syringe,
placing the syringe holder with the needle in front of the injection port of the bag or in front of the septum of the bottle,
moving the syringe holder and the bag or bottle holder towards one another until the injection port or the septum of the bottle is penetrated by the needle on the syringe, and
expelling the drug from the barrel of the syringe by the actuator depressing the plunger of the syringe, thereby injecting the drug into the bag or bottle.

4. A method according to claim 3, further comprising the steps of: recording the weight of the syringe before and after the aspiration of the drug in a computer, and the computer determining the weight difference, the computer determining the drug volume corresponding to the determined weight difference and the computer comparing the determined drug volume with the prescribed volume.

5. A holder for grasping a cap of a vial, comprising:
a housing with a substantially cylindrical recess for receiving the top of the vial, the bottom of said recess being provided with an aperture for allowing needle access to the top of a vial received inside the recess,
three or more radially movable clamping members for engaging the top of the vial, said clamping members being circularly arranged around said recess,
means for urging the clamping members toward the axis of said cylindrical recess, and
means for moving said clamping members away from the axis of said cylindrical recess.

6. An actuator for a vial holder comprising:
a linear drive with a movable drive member,
a vial holder with clamping means for engaging a top of a vial, said vial holder being suspended from said drive member, and
a dynamic weighing cell disposed between the drive member and the vial holder.

7. A receiver for receiving the tip of a Luer-lock syringe, said receiver comprising a funnel shaped housing with a conical section leading to a substantially cylindrical section with a diameter substantially corresponding to a Luer-lock on a syringe.

8. A syringe holder comprising two parallel rims provided with opposing pairs of slits and/or recesses for receiving the flanges at the end of the barrel of the syringe.

9. A holder for holding the free end of the plunger of a syringe, said holder comprising a T-shaped slit, formed by a transverse slit for receiving a part of the thumbplate of the plunger and an axial slit for receiving a part of one of the four rims that make up the plunger rod.

10. A tray for holding an infusion bag, said tray comprising means for holding the injection port of infusion bag, an actuator for effecting a translative movement of the tray relative to a base of the tray in the direction of the injection port and a dynamic weighing cell disposed between the base and the tray.
